# EUROPEAN PATENT SPECIFICATION

(11) **EP 2 789 333 B1**
(45) Date of publication and mention of the grant of the patent: **15.05.2019**
(21) Application number: 12854817.9
(22) Date of filing: 05.12.2012
(51) Int. Cl.: A61K 31/138, A61P 17/00

(54) **APPLICATION OF FLUOXETINE TO TREATMENT OF DEPIGMENTATION DISEASES**
VERWENDUNG VON FLUOXETIN ZUR BEHANDLUNG VON DEPIGMENTIERUNGSERKRANKUNGEN
APPLICATION DE LA FLUOXÉTINE AU TRAITEMENT DE MALADIES DE DÉPIGMENTATION

(30) Priority: 07.12.2011 CN 201110403173
(43) Date of publication of application: 15.10.2014
(73) Proprietor: Shandong Runze Pharmaceuticals Co., Ltd., Heze City Shandong Province 274000 (CN)
(72) Inventor: SHANG, Jing, Nanjing Jiangsu 210009 (CN); LIAO, Sha, Nanjing Jiangsu 210009 (CN); JIN, Yu, Nanjing Jiangsu 210009 (CN); TIAN, Xiaoli, Nanjing Jiangsu 210009 (CN); ZHAO, Guorui, Nanjing Jiangsu 210009 (CN); ZHOU, Jia, Nanjing Jiangsu 210009 (CN); WANG, Qian, Nanjing Jiangsu 210009 (CN); CHONG, Silin, Nanjing Jiangsu 210009 (CN)
(74) Representative: Engelhard, Markus
(86) International application number: PCT/CN2012/085929
(87) International publication number: WO 2013/083040

(56) References cited:
- WO-A1-2008/078353
- CN-A- 101 668 526
- CN-A- 102 429 894

## Description

### BACKGROUND

### Technical Field

The present invention relates to the field of medicine, and particularly relates to the application of fluoxetine to the treatment of depigmentation diseases.

### Related Art

Depigmentation skin disease is a class of common acquired hypopigmentative skin disease caused by the deficiency of skin melanocytes or the reduction and impairment of melanin synthesis function. For example, leukotrichia is a common depigmentation disease involving a wide range of people. Another problematic skin disease is vitiligo, and such a skin disease has occurred throughout the world and can involve all nations, which seriously affects the patient's normal life. Currently, depigmentation skin disease is still difficult to treat and has a high relapse rate. However, the effect of the existing drugs themselves for increasing pigment on promoting pigment synthesis is not significant, and the externally applied topical drugs (e.g., Psoralen) for increasing pigment commonly used in clinic are often difficult to effectively play the role and have photosensitivity. Therefore, there is an increasingly urgent demand for the development of therapeutic drug having definite efficacy.

WO 2008/078353 A1 discloses a use of antidepressant compounds and related compositions for regenerating the integumentary system and/or for stimulating the growth, the original trophism and/or original pigmentation of the corresponding cutaneous appendages, particularly hair.

Fluoxetine, with a chemical name of N-methyl-γ-[4-(trifluoromethyl)phenoxy] phenylpropylamine, and what is used in clinic is fluoxetine hydrochloride, with a trade name of prozac (Prozac®), which is developed by U.S. Eli Lilly and Company, firstly marketed in 1987 in the United States, successively applied in England, France, Germany, Japan, and other countries, and registered in China in 1996 (X960445). Its molecular formula is C₁₇H₁₈F₃NO·HCl and has a molecular weight of 345.79; and its hydrochloride is a white crystal and its melting point is 179°C to 182°C (decomposed).

The main pharmacological effect of fluoxetine lies in that it selectively inhibits the reuptake of 5-serotonin by the presynaptic membrane of the central nervous system. Therefore, it is also called as selective 5-serotonin reuptake inhibitor. Fluoxetine is well absorbed after being orally taken, and its absorption is not affected by foods. Its blood concentration reaches a peak 6 to 8 h after taking, and the half-life of its active metabolite norfluoxetine is 7 to 10 d. Renal excretion is the main route of elimination: about 80% of the drug is excreted in the urine and 15% of the drug is excreted in the stool. Fluoxetine is substantially metabolized in liver, and the liver disease can affect its elimination. It is used in clinic for adults for the treatment of depression, obsessive-compulsive disorder and bulimia nervosa, and also used for the treatment of panic disorder complicated with or without agoraphobia. Fluoxetine mainly selectively acts on 5-serotonin system, and its action on cholinergic system, adrenergic system and histamine system is very weak. Therefore, as compared with the traditional antidepressants such as tricyclic antidepressants, heterocyclic antidepressants, and monoamine oxidase inhibitors, fluoxetine is characterized by good efficacy, weak and less adverse effects, high safety, and good tolerance. Its common adverse effects are gastrointestinal discomfort and neurological disorders, such as anorexia, nausea, headache, insomnia, sweating, and the like. Although fluoxetine is widely used in clinic and has few side effects, its effects on skin pigment synthesis have never been studied.

### SUMMARY

The present invention discloses a therapeutic application of fluoxetine, that is, the application of fluoxetine to the treatment of a depigmentation diseases, wherein the depigmentation disease is leukotrichia or vitiligo.

The followings are a part of the pharmacodynamic tests and results thereof in the present invention:

### Part I: Effects of fluoxetine on melanin synthesis of B16F10 mouse melanoma cell line.

### 1. Effects of fluoxetine on B16F10 cell proliferation

### MTT assay for measuring cell proliferation rate:

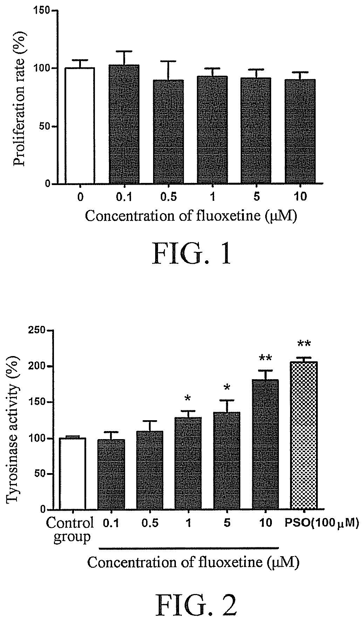

B16F10 cells in the exponential growth phase in good condition were taken, digested, and counted. The cells were resuspended in a high glucose DMEM culture solution containing 10% fetal bovine serum. The cells were inoculated in a 96-well culture plate with an inoculum density of 2.2 × 10⁴ cells/ml and an inoculum size of 180 µl/well, placed in a 5% CO₂ incubator at 37°C, and incubated for 24 hours; fluoxetine hydrochloride with different concentrations was added at 20 µl/ well, and incubated for 72 hours; 20 µl MTT per well was added, allowing reaction in an incubator at 37°C for 4 hours; supernatant was sucked and discarded, 150 µl DMSO per well was added, the plate was shaken on a shaker for 10 minutes; the absorbance of each well was measured at a wavelength of 570 nm using a microwell plate reader, and the cell proliferation rate was calculated. The results were shown in FIG. 1.

Experimental results: compared with the blank control group, fluoxetine administration group (0.1 to 10 µM) had no significant effect on the growth of B16F10 cells (P > 0.05).

Experimental conclusions: fluoxetine (0.1 to 10 µM) showed low toxicity and low carcinogenesis on B16F10 melanoma cells.

### 2. Effects of fluoxetine on tyrosinase activity of B16F10 cells.

### L-DOPA oxidation assay for measuring tyrosinase activity:

B16F10 cells in the exponential growth phase in good condition were taken, digested, and counted. The cells were inoculated in a 6-well plate with an inoculum concentration of 1 × 10⁵ cells/ml and an inoculum size of 2 ml/well, placed in a 5% CO₂ incubator at 37°C and incubated for 24 hours; high glucose DMEM medium containing 2.5% fetal bovine serum was used at 1.8 ml/well, fluoxetine hydrochloride with different concentrations was added at 0.2 ml/well and incubated for 72 hours; washed with PBS twice, cells were collected in doff tubes, 100 µl non-denaturing lysis solution (containing 1 nM PM SF) was added to each tube, lysed at 4°C for 20 min, centrifuged at 4°C, at 12000 r/min for 10 min, the supernatant was taken for protein quantification (BCA method), and the protein concentration was calculated; a amount containing 30 µg protein was taken and added to a 96-well plate, added up to 100 µl with PBS (0.1 M, pH 6.8), then 0.01% L-DOPA 100 µl was added, with three independent wells for each concentration, incubated away from light at 37°C for 60 min, and OD values were measured at a wavelength of 475 nm. The absorbance values of per µg of protein were calculated, with the results expressed in percentage. The results were shown in FIG. 2. Compared with the control group in FIG. 2, * P < 0.05 and ** P < 0.01. PSO is psoralen.

Experimental results: compared with the blank control group, fluoxetine significantly promoted the tyrosinase activity of B16F10 cells; P < 0.05 when fluoxetine was at 1 µM and 5 µM; P < 0.01 when fluoxetine was at 10 µM; and the enzyme activity increase rate of fluoxetine was close to that of the positive drug psoralen when fluoxetine was in the highest concentration.

Experimental conclusions: fluoxetine had significant effects of promoting the activity of tyrosinase which is the rate-limiting enzyme for the melanin synthesis of B16F10 melanoma cells.

### 3. Effects of fluoxetine on the content of melanin of B16F10 cells.

### NaOH lysis method for the determination of melanin content:

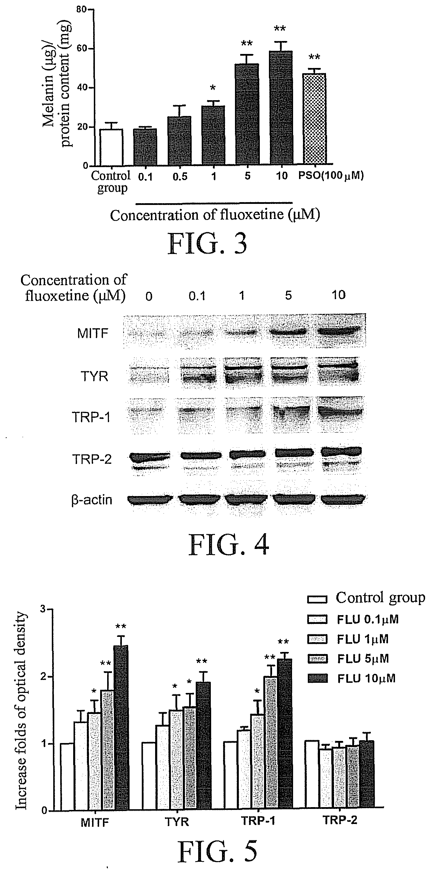

B16F10 cells in the exponential growth phase in good condition were taken, digested, and counted. The cells were inoculated in a Petri dish with a diameter of 10 cm in an inoculum size of 3 × 10⁵ cells/dish, placed in 5% CO₂ incubator at 37°C and incubated for 24 hours; a high glucose DMEM medium containing 2.5% fetal bovine serum was used, fluoxetine hydrochloride with different concentrations was added, incubated for 72 hours; washed with PBS twice, cells were collected, 300 µl non-denaturing lysis solution (containing 1 nM PM SF) was added, lysed at 4°C for 20 min, centrifuged at 4°C, at 12000 r/min for 10 min, the supernatant was taken for protein quantification (BCA method), and the total protein content was calculated; 200 µl NaOH (containing 10% DMSO) was added to the lower layer of melanin pellets, placed in a water bath tank at 80°C and lysed for two hours; a melanin content standard curve was plotted by using the melanin standard; 200 µl/well melanin which was completely dissolved was added to a 96-well plate, the absorbance values at 405 nm were measured, and the melanin content for per mg of protein was calculated. The results were shown in FIG. 3. Compared with the control group in FIG. 3, * P < 0.05, **P < 0.01. PSO is psoralen.

Experimental results: compared with the blank control group, fluoxetine (1 to 10 µM) significantly increased the melanin content of B16F10 cells; P < 0.05 when fluoxetine was at 1 µM and P < 0.01 when fluoxetine was at 5 µM and 10 µM; and the increase rate exceeded that of the positive drug psoralen.

Experimental conclusions: fluoxetine can significantly increase the melanin content of B16F10 cells.

### 4. Effects of fluoxetine on the expression of proteins TYR, TRP-1, TRP-2 and MITF which are important for the melanin synthesis of B16F10 cells.

### Western blotting assay for detecting the expression of proteins important for the melanin synthesis:

B16F10 cells in the exponential growth phase in good condition were taken, digested, and counted. The cells were inoculated in a 6-well plate, grouped as above for drug treatment, cells were collected after 72 h and cell lysis solution was added, centrifuged, the supernatant was taken, the protein concentration was determined by BCA method, the protein lysis solution was adjusted to the same concentration, mixed uniformly with twice of sample-loading buffer with equal volume, boiled for 5 min, and SDS-PAGE polyacrylamide gel electrophoresis was carried out; after the electrophoresis was completed, the protein was transferred to a NC membrane with a voltage of 100 V for 1 h; blocked with TBST (0.05% Tween-20) containing 5% skimmed milk for 1 h; the membrane was incubated at 4°C overnight respectively with primary antibodies (1:200) against TYR, TRP-1, TRP-2, MITF and β-actin which were dissolved in a blocking solution, washed with TBST 3 times with 5 min for each time; subsequently the corresponding secondary antibodies (1:4000) were added and incubated for 1 h, washed 3 times with 5 min for each time; the membrane was immersed in a prepared ECL supersensitive light-sensitive mixed liquid, with timing for 5 min, X-ray films were taken in a darkroom and the membrane was compressed to emit light; the results were obtained by developing and photographic fixing, and the results were analyzed using Quantity One Software from Bio-Rad company. The results were shown in FIG. 4 and FIG. 5. Compared with the control group in the figures, *P < 0.05 and **P < 0.01. FLU represents fluoxetine.

Experimental results: compared with the blank control group, fluoxetine can significantly improve the expression of proteins MITF, tyrosinase (TYR) and tyrosinase-related protein 1 (TRP-1) of B16F10 cells, and there was statistically significant difference. However, the protein level of tyrosinase-related protein 2 (TRP-2) was not significantly affected.

Experimental conclusions: fluoxetine can increase the melanin content through improving the expression of proteins (MITF, TYR, TRP1) which are important for the melanin synthesis of B16F10 cells.

### Part II: Effects of fluoxetine on the melanin synthesis of normal human primary melanocytes

### 1. Effects of fluoxetine on the proliferation of human primary melanocytes

### MTT assay for measuring cell proliferation rate:

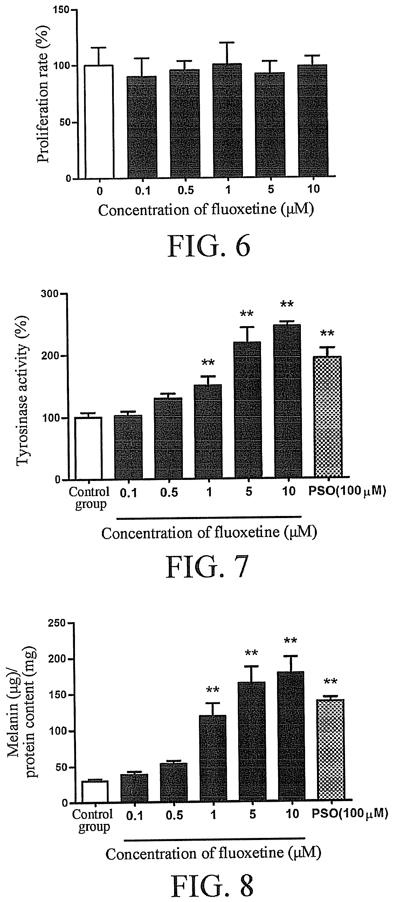

Normal human primary melanocytes in the exponential growth phase in good condition were taken, digested, and counted. The cells were inoculated in a 96-well culture plate with an inoculum density of 5 × 10⁴ cells/ml and an inoculum size of 180 µl/well, placed in a 5% CO₂ incubator at 37°C, and incubated for 24 hours; fluoxetine hydrochloride with different concentrations was added and incubated for 72 hours; 20 µl MTT per well was added, allowing reaction in an incubator at 37°C for 4 hours; supernatant was sucked and discarded, 150 µl DMSO per well was added, the plate was shaken on a shaker for 10 minutes; the absorbance of each well was measured at a wavelength of 570 nm using a microwell plate reader, and the cell proliferation rate was calculated. The results were shown in FIG. 6.

Experimental results: compared with the blank control group, fluoxetine administration group had no significant effect on the growth of normal human primary melanocytes (P > 0.05).

Experimental conclusions: fluoxetine had no significant effect on the proliferation of human primary melanocytes and showed relatively low toxicity.

### 2. Effects of fluoxetine on tyrosinase activity of human primary melanocytes

### L-DOPA oxidation assay for determination of tyrosinase activity:

Human primary melanocytes in the exponential growth phase in good condition were taken, digested, and counted. The cells were inoculated in a 6-well plate with an inoculum concentration of 1 × 10⁵ cells/ml and an inoculum size of 2 ml/well, placed in a 5% CO₂ incubator at 37°C, and incubated for 24 hours; fluoxetine hydrochloride with different concentrations was added and incubated for 72 hours, washed with PBS twice, cells were collected in doff tubes, 80 µl non-denaturing lysis solution (containing 1 nM PM SF) was added to each tube, lysed at 4°C for 20 min, centrifuged at 4°C and 12000 r/min for 10 min, the supernatant was taken for protein quantification (BCA method), and the protein concentration was calculated; protein having a volume containing 10 µg was taken and added to a 96-well plate, added up to 100 µl with PBS (0.1 M, pH 6.8), then 0.01% L-DOPA 100 µl was added, with three wells for each concentration, incubated away from light at 37°C for 60 min, and OD values were measured at a wavelength of 475 nm. The absorbance values of per µg of protein were calculated, with the results expressed in percentage. The results were shown in FIG. 7. Compared with the control group in FIG. 7, *P < 0.05 and **P < 0.01. PSO represents psoralen.

Experimental results: compared with the blank control group, fluoxetine significantly promoted the tyrosinase activity of human primary melanocytes (P < 0.01); and the enzyme activity increase rate exceeded that of the positive drug psoralen when fluoxetine was at 5 µM and 10 µM.

Experimental conclusions: fluoxetine within the tested concentrations had significant effects of promoting the activity of tyrosinase which is the rate-limiting enzyme for the melanin synthesis of human primary melanocytes.

### 3. Effects of fluoxetine on the melanin content of human primary melanocytes

### NaOH lysis method for measuring melanin content:

Human primary melanocytes in the exponential growth phase in good condition were taken, digested, and counted. The cells were inoculated in a 6-well plate with an inoculum concentration of 1 × 10⁵ cells/ml and an inoculum size of 2 ml/well, placed in 5% CO₂ incubator at 37°C and incubated for 24 hours; fluoxetine hydrochloride with different concentrations was added, and incubated for 72 hours; washed with PBS twice, cells were collected, 80 µl non-denaturing lysis solution (containing 1 nM PM SF) was added, lysed at 4°C for 20 min, centrifuged at 4°C, at 12000 r/min for 10 min, the supernatant was taken for protein quantification (BCA method), and the total protein content was calculated; 100 µl NaOH (containing 10% DMSO) was added to the lower layer of melanin pellets, placed in a water bath tank at 80°C and lysed for two hours; a melanin content standard curve was plotted by using the melanin standard; 100 µl/well melanin which was completely dissolved was added to a 96-well plate, the absorbance values at 405 nm were measured, and the melanin content for per mg of protein was calculated. The results were shown in FIG. 8. Compared with the control group in FIG. 8, *P < 0.05 and **P < 0.01. PSO is psoralen.

Experimental results: compared with the control group, fluoxetine significantly increased the melanin content of human primary melanocytes (P < 0.01); and the increase rate was close to or exceeded that of the positive drug psoralen.

Experimental conclusions: fluoxetine can significantly increase the melanin content of human primary melanocytes.

### 4. Effects of fluoxetine on the expression of proteins TYR, TRP-1, TRP-2 and MITF important for the melanin synthesis of human primary melanocytes.

### Western blotting assay for detecting the expression of proteins which are important for the melanin synthesis:

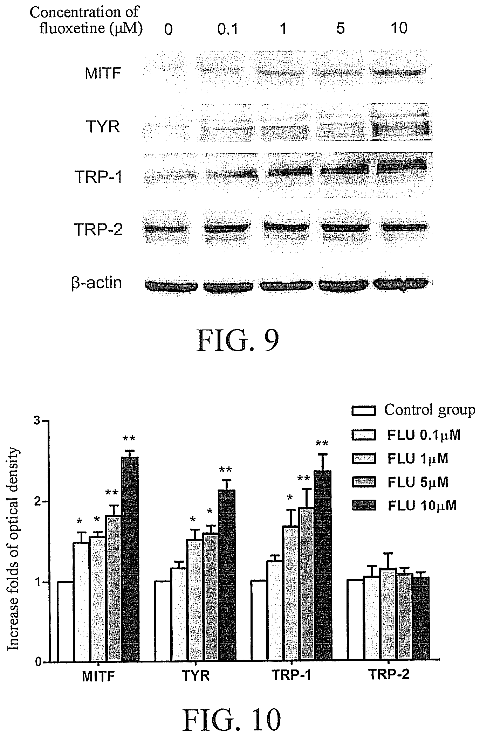

Human primary melanocytes in the exponential growth phase in good condition were taken, digested, and counted. The cells were inoculated in a 6-well plate, grouped as above for drug treatment, cells were collected after 72 h and cell lysis solution was added, centrifuged, the supernatant was taken, the protein concentration was determined by BCA method, the protein lysis solution was adjusted to the same concentration, mixed uniformly with twice of sample-loading buffer with equal volume, boiled for 5 min, and SDS-PAGE polyacrylamide gel electrophoresis was carried out; after the electrophoresis was completed, the protein was transferred to a NC membrane with a voltage of 100 V for 1 h, blocked with TBST (0.05% Tween-20) containing 5% skimmed milk for 1 h; the membrane was incubated at 4°C overnight respectively with primary antibodies (1:200) against TYR, TRP-1, TRP-2, MITF and β-actin which are dissolved in blocking solution, washed with TBST 3 times with 5 min for each time; the corresponding secondary antibodies (1:4000) were added and incubated for 1 h, washed with TBST 3 times with 5 min for each time; the membrane was immersed in a prepared ECL supersensitive light-sensitive mixed liquid, with timing for 5 min, X-ray films were taken in a darkroom and the membrane was compressed to emit light; the results were obtained by developing and photographic fixing, and the results were analyzed using Quantity One Software from Bio-Rad company. The results were shown in FIG. 9 and FIG. 10. Compared with the control group, *P < 0.05 and **P < 0.01. FLU represents fluoxetine.

Experimental results: compared with the blank control group, fluoxetine can significantly improve the expression of proteins MITF, tyrosinase (TYR) and tyrosinase-related protein 1 (TRP-1) of human primary melanocytes, and there was statistically significant difference. However, the protein level of tyrosinase-related protein 2 (TRP-2) was not significantly affected.

Experimental conclusions: fluoxetine can increase the melanin content through improving the expression of proteins (MITF, TYR, TRP-1) which are important for the melanin synthesis of human primary melanocytes.

### Part III: Effects of fluoxetine on the expression of hair follicle melanin and melanin synthesis protein in hair-removed synchronized C57BL/ 6 mice.

### 1. Effects of fluoxetine on melanin synthesis in skin hair follicle of C57BL/6 mice

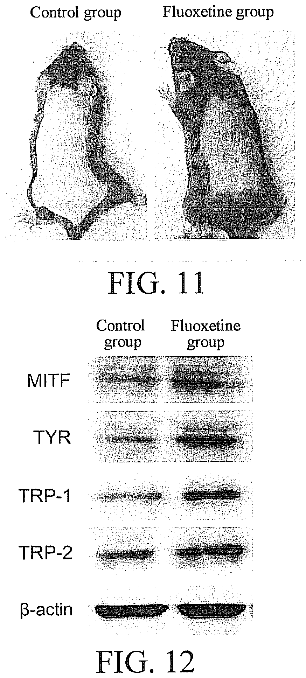

C57BL/6 mice was fed for acclimation, and the back hair was removed with a rosin/paraffin mixture 1 day before the experiment, to induce the synchronous growth cycle of the hair follicles. Animals were randomly divided into a solvent control group (physiological saline) and a fluoxetine administration group (20 mg/kg), with 10 animals for each group. Reference to clinical administration method was made, and gavage was performed once a day for consecutive 12 days. On the 12^{th} day after administration, the condition about the skin color on the back of the mice was recorded by photographing. The results were shown in FIG. 11.

Experimental results: the pictures about the skin on the back of the mice on the 12^{th} day after administration showed that the skin color in the hair-removed area of the skin of the fluoxetine administration group was significantly darkened as compared with that of the solvent control group.

Experimental conclusions: fluoxetine gavage for 12 days can significantly promote the melanin synthesis in the skin of hair-removed synchronized C57BL/6 mice.

### 2. Western blotting assay for detecting the expression of proteins important for melanin synthesis in hair follicles of C57BL/6 mice

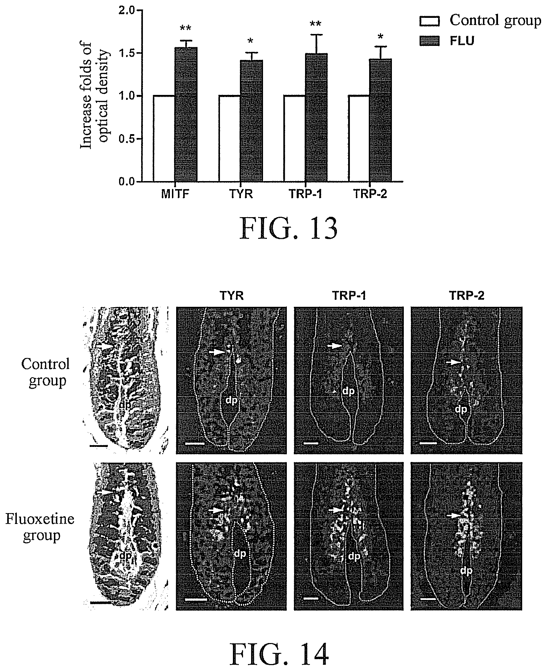

On the 12^{th} day after administration, the above animals were sacrificed to collect skin samples for Western blotting experiment. A certain amount of skin tissue of C57BL/6 mice was taken and sheared into pieces. A tissue lysis solution and a proteinase inhibitor were added. Protein was extracted after homogenization; the protein concentration was determined by BCA method, the protein lysis buffer solution was adjusted to the same concentration, mixed uniformly with twice of sample-loading buffer with equal volume, boiled for 5 min, and SDS-PAGE polyacrylamide gel electrophoresis was carried out; after the electrophoresis was completed, the protein was transferred to a NC membrane with a voltage of 100 V for 1 h, blocked with TBST (0.05% Tween-20) containing 5% skimmed milk for 1 h; the membrane was incubated at 4°C overnight respectively with primary antibodies (1:200) against TYR, TRP-1, TRP-2, MITF and β-actin which are dissolved in blocking solution, washed with TBST 3 times with 5 min for each time; subsequently the corresponding secondary antibodies (1:4000) were added and incubated for 1 h, washed 3 times with 5 min for each time; the membrane was immersed in a prepared ECL supersensitive light-sensitive mixed liquid, with timing for 5 min, X-ray films were taken in a darkroom and the membrane was compressed to emit light; the results were obtained by developing and photographic fixing, and the results were analyzed using Quantity One Software from Bio-Rad company. The results were shown in FIG. 12 and FIG. 13. Compared with the control group in the figures, *P < 0.05 and **P < 0.01. FLU represents fluoxetine group.

Experimental results: compared with the blank control group, fluoxetine can significantly improve the expression of proteins MITF, tyrosinase (TYR), tyrosinase-related protein 1 (TRP-1) and tyrosinase-related protein 2 (TRP-2) in hair-removed synchronized skin hair follicles of C57BL/6 mice, and there was statistically significant difference.

Experimental conclusions: fluoxetine can increase the melanin content in hair follicles through improving the expression of proteins which are important for the melanin synthesis in the hair-removed synchronized skin hair follicles melanocytes of C57BL/6 mice.

### 3. Immunohistochemisty assay for detecting the expression of proteins which are important for melanin synthesis in hair follicles of C57BL/6 mice

On the 12^{th} day after administration, the animals were sacrificed, and skin samples were collected parallel to the spine line. Skin tissues were fixed in 10% formaldehyde for 48 h and embedded in paraffin, then sliced longitudinally along the hair follicles (4 µm), and HE staining and immunohistochemical staining were carried out respectively. HE staining: conventional hematoxylin-eosin staining. Immunohistochemical staining: the slices were dewaxed and hydrated, incubated with 3% hydrogen peroxide at room temperature for 10 min, washed with PBS for 5 min and repeated three times. A goat serum blocking solution was added dropwise for blocking for 20 min after heat-induced antigen retrieval, and rabbit-derived TYR, TRP-1 and TRP-2 antibody were added, and incubated at 4°C overnight. TRITC-labeled goat anti-rabbit IgG was added after washing with PBS three times, incubated at 37°C for 30 min and washed with PBS three times with each time for 5 min. An anti-fluorescent quencher was added and the slices were mounted. The diluted solutions of the primary antibodies were used to replace the primary antibodies to repeat the above processes, as a negative control experiment. Two skin slices were selected for each animal and five visual fields were randomly selected for each slice for photographing and recording with a fluorescence microscope. The results were shown in FIG. 14. The scale in the pictures are: 20 µm; dp, hair papilla.

Experimental results: as compared with the blank control group, fluoxetine can significantly improve the expression of proteins tyrosinase (TYR), tyrosinase-related protein 1 (TRP-1) and tyrosinase-related protein 2 (TRP-2) in the hair follicle melanocytes in hair-removed synchronized skin areas located above the hair papilla of C57BL/6 mice.

Experimental conclusions: fluoxetine can increase the hair follicle melanin content through improving the expression of proteins which are important for the melanin synthesis in the hair follicle melanocytes in hair-removed synchronized skin of C57BL/6 mice, which is identical with the Western Blotting result.

### BRIEF DESCRIPTION OF THE DRAWINGS

The disclosure will become more fully understood from the detailed description given herein below for illustration only, and thus are not limitative of the disclosure, and wherein:
FIG. 1 shows the effects of fluoxetine on the growth of B16F10 cells;
FIG. 2 shows the effects of fluoxetine on tyrosinase activity of B16F10 cells;
FIG. 3 shows the effects of fluoxetine on the content of melanin of B16F10 cells;
FIG. 4 shows the effects of fluoxetine on the expression of proteins MITF, TYR, TRP-1, and TRP-2 which are important for the melanin synthesis of B16F10 cells;
FIG. 5 shows the scanning results for the gray scales of bands with fluoxetine for the proteins MITF, TYR, TRP-1, and TRP-2 which are important for the melanin synthesis of B16F10 cells;
FIG. 6 shows the effects of fluoxetine on the proliferation of human primary melanocytes;
FIG. 7 shows the effects of fluoxetine on tyrosinase activity of human primary melanocytes;
FIG. 8 shows the effects of fluoxetine on the melanin content of human primary melanocytes;
FIG. 9 shows the effects of fluoxetine on the expression of proteins MITF, TYR, TRP-1, and TRP-2 which are important for the melanin synthesis of human primary melanocytes;
FIG. 10 shows the scanning results for the gray scales of bands with fluoxetine for the proteins MITF, TYR, TRP-1, and TRP-2 which are important for the melanin synthesis of human primary melanocytes;
FIG. 11 shows the condition about the skin color on the back of the hair-removed synchronized C57BL/6 mice on the 12th day after administration;
FIG. 12 shows the effects of fluoxetine on the expression of proteins MITF, TYR, TRP-1, and TRP-2 in hair follicle melanocytes of C57BL/6 mice;
FIG. 13 shows the scanning results for the gray scales of bands with fluoxetine for MITF, TYR, TRP-1 and TRP-2 in hair follicle melanocytes of C57BL/6 mice; and
FIG. 14 shows the immunohistochemical results for the proteins which are important for the synthesis of hair follicle melanin of C57BL/6 mice.

## Claims

1. Fluoxetine for use in the treatment and/or prevention of a depigmentation disease, wherein the depigmentation disease is vitiligo or leukotrichia.

## Patentansprüche

1. Fluoxetin zur Verwendung bei der Behandlung und/oder Verhinderung einer Depigmentierungskrankheit, wobei die Depigmentierungskrankheit Vitiligo oder Leukotrichose ist.

## Revendications

1. Fluoxétine pour une utilisation dans le traitement et/ou la prévention d'une maladie dépigmentante, dans laquelle la maladie dépigmentante est le vitiligo ou la leucotrichie.
